(19) Europäisches Patentamt
European Patent Office
Office européen des brevets

(11) Veröffentlichungsnummer: **0 054 952**
**B1**

(12) # EUROPÄISCHE PATENTSCHRIFT

(45) Veröffentlichungstag der Patentschrift: **10.10.84**

(51) Int. Cl.³: **G 01 N 33/54,** G 01 N 33/58

(21) Anmeldenummer: **81110656.6**

(22) Anmeldetag: **21.12.81**

(54) **Verfahren zur Bestimmung von Antigenen, Antikörpern und deren Komplexen.**

(30) Priorität: **22.12.80 DE 3048477**
**20.02.81 DE 3106444**
**10.08.81 DE 3131615**
**02.11.81 DE 3143423**

(43) Veröffentlichungstag der Anmeldung:
**30.06.82 Patentblatt 82/26**

(45) Bekanntmachung des Hinweises auf die Patenterteilung:
**10.10.84 Patentblatt 84/41**

(84) Benannte Vertragsstaaten:
**AT BE CH FR GB IT LI NL SE**

(56) Entgegenhaltungen:
**US - A - 4 193 983**
**US - A - 4 238 195**

**THE JOURNAL OF PHYSICAL CHEMISTRY, Band 78, Nr. 17, August 1974, Easton (US), R. NILSSON et al. "Role of Singlet Oxygen in Some Chemiluminescence and Enzyme Oxidation Reactions", Seiten 1681 bis 1683**
**THE JOURNAL OF IMMUNOLOGY, Band 95, Nr. 2, 1965, Baltimore, B.T. WOOD et al., "Fluorescent Antibody Staining", Seiten 225 bis 229**
**JOURNAL OF IMMUNOLOGICAL METHODS, Band 21,**

(73) Patentinhaber: **Seromed medizinische Vertriebs GmbH, Paulsborner Strasse 3, D-1000 Berlin 31 (DE)**
Patentinhaber: **Frenzel, Bernd, Dr., Spitzelbergstrasse 18a, D-8000 München 71 (DE)**

(72) Erfinder: **Frenzel, Bernd, Dr., Spitzelbergstrasse 18a, D-8000 München 71 (DE)**

(74) Vertreter: **Hagemann, Heinrich, Dr. et al, Patentanwälte GEYER, HAGEMANN & PARTNER Postfach 860329, D-8000 München 86 (DE)**

(56) Entgegenhaltungen: (Fortsetzung)
**1978, J.J. PRATT et al. "Chemiluminescence-linked Immunoassay", Seiten 179 bis 184**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 054 952 B1

BUNDESDRUCKEREI BERLIN

**Beschreibung**

Die Erfindung bezieht sich auf ein Verfahren zur quantitativen und qualitativen Bestimmung von Antigenen, Antikörpern und deren Komplexen mittels einer chemolumineszierenden Markierungssubstanz und einem Anregungsmittel hierfür unter Anwendung an sich bekannter heterogener Assays.

Es ist von großer Bedeutung, Antigene, Antikörper oder deren Komplexe in Sekreten, Exkreten und Körperflüssigkeiten von Wirbeltierorganismen sowie von Menschen zu messen. Auf diese Weise können u. a. diagnostische Aussagen gemacht werden.

Es ist bekannt, eine serologische Reaktion durch Markierung einer oder mehrerer Reaktionskomponenten mit einem radioaktiven Isotop, durch Konjugierung mit einem Enzym, einem Fluoreszenzfarbstoff oder einer chemolumineszierenden Substanz, wie Luminol oder Luciferin, nachzuweisen.

Das Radioimmunoassay wird in Journal Clinical Endocrinology 27 (1967), S. 973 und Ibid 28 (1968), S. 343, beschrieben. Der wesentliche Nachteil dieses Assays liegt in dem notwendigen Umgang mit strahlenemittierenden Isotopen und in der erforderlichen aufwendigen Ausrüstung zur Durchführung eines solchen Assays.

Bei der Markierung einer Reaktionskomponente mit einem Enzym besteht der Nachteil darin, daß diese Markierung kompliziert durchzuführen und das hergestellte Reaktionsprodukt schwierig aufzubewahren und zu gebrauchen ist. Darüber hinaus handelt es sich bei den einzusetzenden Enzymen um biologisch aktive Substanzen extrem komplexer Natur, auf die die erwähnten Schwierigkeiten zurückgehen. Die schließlich zum Nachweis des gebundenen Enzyms einzusetzenden Substrate sind darüber hinaus noch kanzerogen. Das ist nachteilig. Das Enzym-Assay der beschriebenen Art wird in Bull. World Health Organ 53 (1976) abgehandelt.

Bei der Fluoreszenztechnik wird ein Antigene und Antikörper enthaltendes Reaktionsprodukt durch Fluoreszenz unter Bestrahlung mit kurzwelligem Licht nachgewiesen. Hierbei muß das Anregungslicht vom emittierten Licht nachteiligerweise mit großem, apparativem Aufwand abgetrennt werden.

Bei den bisher zur Chemolumineszenz herangezogenen chemolumineszierenden Substanzen handelt es sich um solche, die nur sehr schwer an die Reaktionspartner einer serologischen Reaktion zu binden sind und darüber hinaus bis zu 99,3% ihrer ursprünglichen Lumineszenz nach der Bindung verlieren. Das ergibt sich z. B. aus Nature, Vol. 299 (1979), S. 646—647. Des weiteren wird in Journal of Immunological Methods 21 (1978), S. 178—184, darüber berichtet, daß das chemolumineszierende Luminol aus diesem Grunde für klinische Routinelabortests ungeeignet ist.

Dem stehen Angaben in der US-PS 4 193 983 entgegen. Dort wird gerade Luminol als eine besonders geeignete Verbindung für diesen Zweck angegeben. Als Anregungsmittel für die Chemolumineszenz sollen Wasserstoffperoxid oder Calciumhypochlorit in Betracht kommen. Des weiteren wird in dieser US-Patentschrift Fluoreszeinisothiocyanat für ein homogenes Fluoreszenz-Assay erwähnt.

Trotz intensiver Bemühungen war es bisher nicht möglich, ein Verfahren der eingangs beschriebenen Art bezüglich Aufwand und Effizienz zufriedenstellend durchzuführen. Das gilt zum Beispiel auch für das in der US-PS 4 238 195 beschriebene Assay. Dort werden Markierungssubstanzen in Form von Fluoreszein und dessen Derivaten vorgeschlagen, die mittels besonders energiereicher Produkte angeregt werden sollen. Diese Produkte werden durch eine sehr komplexe Reaktion zwischen Oxalsäurederivaten, u. a. Oxalylchlorid, und Wasserstoffperoxid hergestellt. Ihre Herstellung erfordert einen hohen Arbeitsaufwand. Darüber hinaus sind derartige Produkte nicht unbedenklich, da sie unter Umständen das giftige Phosgen entwickeln können.

Der Erfindung liegt die Aufgabe zugrunde, aus der Vielzahl der bekannten chemolumineszierenden Markierungssubstanzen und Anregungsmittel solche Kombinationen derselben aufzufinden, die es gestatten, das eingangs beschriebene Verfahren so zu verbessern, daß es gefahrlos, einfach und mit hoher Effizienz durchführbar ist.

Erfindungsgemäß wird diese Aufgabe dadurch gelöst, daß eine der folgenden Kombinationen aus Anregungsmittel und Markierungssubstanz verwendet wird:

$H_2O_2$/Chloramin bzw. -derivat und Fluoreszein bzw. -derivat,
$H_2O_2$/Chloramin bzw. -derivat und Methylenblau bzw. -derivat,
$H_2O_2$/Chloramin bzw. -derivat und Thionin bzw. -derivat,
Calciumhypochlorit und Fluoreszein bzw. -derivat.

Wenn im Zusammenhang mit der Erfindung von Antigenen und Antikörpern gesprochen wird, so sollen diese Begriffe weitestgehend verstanden werden. Bei Antigenen handelt es sich um Stoffe, die nach Einführung in den Organismus von Menschen und Tieren die Bildung von Antikörpern hervorrufen. Als Antigene wirken artfremde Eiweißstoffe tierischer und pflanzlicher Herkunft, besonders diejenigen von Infektionserregern, sowie viele Stoffe komplizierter Natur mit fett-, saccharid- (bzw. zucker-), amin- und azoartiger Struktur. So kann es sich dabei um Substanzen, z. B. Proteide, Proteine, Polysaccharide, Lipide oder Nucleinsäuren handeln, die im Organismus von Wirbeltieren sowie von Menschen die Bildung von Antikörpern hervorrufen und spezifisch mit diesen reagieren. Auch sollen hierzu ganz allgemein Haptene gezählt werden, zu denen man auch »unvollständige Antigene« sagt, die wegen ihrer geringen Größe allein keine Bildung von Antikörpern bewirken, jedoch mit den

entsprechenden Antikörpern eine spezifische Bindung eingehen können. Antigene können z. B. Vieren, Bakterien oder Pilze oder Teile von diesen sein. Darüber hinaus sind z. B. auch bestimmte Hormone, Vitamine, Enzyme oder bestimmte Medikamente dem Begriff »Antigen« unterzuordnen. Zur Definition der Begriffe »Antigene« und »Antikörper« sei auf Kabat »Einführung in die Immunchemie und Immunologie«, Springer Verlag, 1971, S. 9—25 und S. 143—197 verwiesen.

Im Sinne der Erfindung handelt es sich bei Antikörpern um spezifische Produkte der Immunantwort, die im Wirbeltier- bzw. im menschlichen Organismus nach einem Antigenkontakt gebildet werden und spezifisch mit dem Antigen reagieren können. Neben die Antikörper sollen ausdrücklich bestimmte Bindeproteine gestellt werden, die sich spezifisch an ein oder mehrere Substanzen, wie z. B. Antikörper, binden können. Ein Beispiel hierfür ist das Protein A. Von besonderer Bedeutung sind die Antikörper, die den Immunglobulinen der Klasse IgG, IgM, IgA und IgE unterzuordnen sind. Diese werden im einzelnen in Kabat »Einführung in die Immunchemie und Immunologie«, Springer Verlag, 1971, S. 143—197, beschrieben.

Von besonderer Bedeutung ist das erfindungsgemäße Verfahren zum Nachweis von Antigenen und Antikörpern bei viralen und bakteriellen Erkrankungen. Dabei ist der Nachweis des Oberflächenantigens des Hepatitis-B-Virus von ganz besonderer Bedeutung. Mit besonderem Vorteil gelingt auch der Nachweis von Antikörpern gegen die Herpes- oder Toga-Viren.

Bei der Verwendung von Fluoreszein oder Fluoreszeinderivaten hat es sich gezeigt, daß sie sich besonders gut an Proteine bzw. Proteide binden lassen. Das bedeutet, daß Fluoreszein mit einer oder mehreren Gruppen substituiert ist, die dessen Affinität im Hinblick auf die Kopplung mit den erwähnten Antigenen und Antikörpern begünstigen. Als besonders vorteilhaft haben sich dabei die Isothiocyanat- und Isocyanatgruppe als die Kopplung vermittelnde bzw. begünstigende Substituenten erwiesen. Eine besonders bevorzugte Stellung der Isothiocyanatgruppe ergibt sich aus der nachfolgenden Formel (FITC).

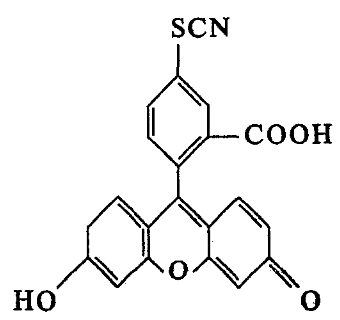

Die Kopplungsfähigkeit des Fluoreszeins, das gegebenenfalls neben den die Kopplung begünstigenden bzw. vermittelnden Substituenten auch noch mit nicht koppelnden Substituenten versehen sein kann, kann hervorgerufen bzw. verbessert werden, indem ein geeignetes Reagens hinzugegeben wird, das eine Kopplungsreaktion bzw. eine Brückenbildung zwischen dem Fluoreszeingrundkörper und dem jeweiligen Antigen bzw. Antikörper oder deren Äquivalente hervorruft.

Der Vorteil bei der Verwendung von Methylenblau liegt darin, daß, insbesondere im basischen Medium, zur Kopplung mit Antigen, Antikörpern oder deren Komplexen keine zusätzliche koppelnde Gruppe erforderlich ist.

Im Falle der Verwendung von Thionin als Markierungssubstanz muß eine koppelnde Gruppe eingeführt werden. Zu dieser koppelnden Gruppe gilt das im Zusammenhang mit Fluoreszein Gesagte entsprechend. In der Praxis hat es sich gezeigt, daß Carbodiimidderivate hierzu besonders geeignet sind, so insbesondere (N-Äthyl-N'-(III-methylamino-propyl)-carbodiimid-Hydrochlorid.

Wenn im Sinne der Erfindung von »Kopplungsfähigkeit« gesprochen wird, so soll auch diese Eigenschaft weitestgehend verstanden werden. In keinem Fall soll damit ausdrücklich auf eine ganz bestimmte Bindungsart abgestellt werden. Vielmehr soll nur zum Ausdruck gebracht werden, daß durch eine Wechselwirkung zwischen der Markierungssubstanz und dem Partner einer serologischen Reaktion in irgendeiner Form ein bindender bzw. ein bindendes komplexartiges Gebilde entsteht.

In Einzelfällen ist es denkbar, daß das zu markierende Antigen nicht oder nicht in ausreichendem Maße mit der jeweils herangezogenen chemolumineszierenden Markierungssubstanz reagieren kann. In solchen Fällen werden dem Fachmann geläufige Proteine oder Proteide verwendet, die zunächst an das Antigen gebunden werden, so daß nachfolgend die Markierungssubstanz an das Protein des entstandenen Reaktionsproduktes gebunden wird. Hierbei kann auch umgekehrt verfahren werden, indem die Markierungssubstanz zunächst an das Protein bzw. Proteid gebunden wird und ein derartiges chemolumineszierendes Konjugat mit dem fraglichen Antigen in Wechselwirkung gebracht wird. Im übrigen hat es sich gezeigt, daß die Empfindlichkeit der vorliegenden Assays ganz beachtlich gesteigert werden kann, wenn bei der Herstellung des chemolumineszieredén Konjugats die chemolumineszierende Markierungssubstanz in Form von Fluoreszein bzw. dessen Derivaten an ein Protein,

insbesondere ein Immunglobolin gebunden wird.

Die erfindungsgemäß einzusetzenden Anregungsmittel reagieren grundsätzlich mit der mit dem Antigen, dem Antikörper oder deren Komplexen gekoppelten chemolumineszierenden Markierungssubstanz in der Weise, daß letztere Photonen aussendet.

Für die erfindungsgemäß herangezogenen chemolumineszierenden Markierungssubstanzen hat sich insbesondere die Kombination aus Wasserstoffperoxid und Chloramin bzw. seinen Derivaten als sehr geeignet erwiesen. Es muß verwundern, daß Wasserstoffperoxid, das in der Literatur häufig ganz allein als einziges Anregungsmittel genannt wird, für die Zwecke der Erfindung allein nicht geeignet ist. Entsprechendes gilt auch für Chloramin bzw. seine Derivate, wenn sie allein eingesetzt werden. Es muß in hohem Maße überraschen, daß die Kombination aus diesen beiden Ausgangsmaterialien zu einer besonders günstigen Lösung der gestellten Aufgabe führt. Unter den in Frage kommenden Chloraminen wird das Chloramin T bevorzugt. Chloramin T ist der internationale Freiname für N-Chlor-4-toluolsulfonsäureamid-Natrium bzw. Tosylchloramid-Natrium. Es handelt sich um eine sehr stabile Substanz, die, wie Calciumhypochlorit, eine besonders gute Reproduzierbarkeit des jeweiligen Assays gewährleistet. Die im Einzelfall eingesetzte Menge des Chloramins (bzw. -derivats) ist mit der Wasserstoffperoxidmenge abzustimmen. Dabei spielt es auch eine Rolle, in welcher Konzentration die Wasserstoffperoxidlösung gewählt wird. So hat es sich gezeigt, daß mit folgenden Konzentrationen vorzügliche Ergebnisse erzielbar sind, wenn gleiche Lösungsvolumina der beiden Reagenzien zusammengegeben werden: eine 0,5- bis 30volumenprozentuale Wasserstoffperoxidlösung und eine 0,1- bis 8gewichtsprozentuale Lösung an Chloramin T. In jedem Fall wird der Fachmann im Rahmen eines geringfügigen Arbeitsaufwandes und ohne erfinderisches Zutun die wirksame Zusammensetzung des Anregungsmittels aus Wasserstoffperoxid und Chloramin bzw. dessen Derivaten ermitteln können.

Calciumhypochlorit, das als Anregungsmittel im Falle der Verwendung von Fluoreszeinisocyanat herangezogen wird, zeigt, insbesondere in wäßriger Lösung, verschiedene beachtliche Vorteile. So läßt sich das Assay außergewöhnlich exakt reproduzieren. Die im Einzelfall gewünschte exakte Konzentration des Anregungsmittels läßt sich ohne weiteres festlegen. Allgemein kann gesagt werden, daß eine etwa 0,1- bis 20gew.-%ige und insbesondere eine 0,2- bis 10gew.-%ige Calciumhypochloritlösung. zu vorteilhaften Ergebnissen führt. Ganz besonders bevorzugt wird eine etwa 1,0- bis 4,0gew.-%ige Lösung, wobei sich eine 2gew.-%ige Lösung als ganz besonders günstig erweist.

Das erfindungsgemäße Verfahren kann, wie bereits einleitend angedeutet, auf der Grundlage an sich bekannter heterogener Assays durchgeführt werden. Derartige Verfahren werden in vielfältiger Weise in der Literatur beschrieben. Hierzu soll zum Beispiel verwiesen werden auf die US-PS 4 238 195 (siehe insbesondere Spalten 7 bis 11, Zeile 15).

Das erfindungsgemäße Verfahren läßt sich demzufolge in vielfältiger Weise, was dem Fachmann ohne weiteres erkennbar ist, verwirklichen. Grundsätzlich muß zunächst, was in an sich bekannter Weise geschieht, der Antigen/Antikörper-Komplex auf Grund einer serologischen Reaktion gebildet werden. Dies kann auf verschiedene Weise erfolgen, indem zunächst entweder das Antigen und der Antikörper in Lösung vorliegen oder ein Partner, d. h. das Antigen oder der Antikörper an einer festen Phase gebunden ist. Hierbei spricht man entweder von einem Flüssigphasen- oder einem Festphasen-Assay. Nach Abschluß der serologischen Reaktion wird in beiden Fällen der Antigen/Antikörper-Komplex von dem verbleibenden Reaktionsmedium abgetrennt. Bei dem Festphasen-Assay kann das in einfacher Weise dadurch geschehen, daß die überstehende Flüssigkeit, gegebenenfalls nach Zentrifugieren, dekantiert wird. Beim Flüssigphasen-Assay erfolgt die Trennung zum Beispiel durch Zentrifugieren mit anschließendem Dekantieren bzw. Filtrieren, insbesondere durch Ultrafiltration, Chromatographieren und dergleichen. Der in der geschilderten oder einer ähnlichen Weise isolierte Antigen/Antikörper-Komplex wird anschließend mit einem flüssigen Medium in Kontakt gebracht, welches das damit in Wechselwirkung zu bringende chemolumineszierende Konjugat enthält.

Das chemolumineszierende Konjugat enthält eine der vorgenannten chemolumineszierenden Markierungssubstanzen neben dem Antigen oder Antikörper (bzw. Protein). Aus dem chemolumineszierenden Konjugat und dem Antigen/Antikörper-Komplex bildet sich ein neuer chemolumineszierender Komplex, der zum Beispiel anhand einer oder mehrerer der vorgenannten Separationsmethoden isoliert wird. Dabei kann er bereits in der Meßküvette vorliegen bzw. darin entstanden sein oder wird in eine solche überführt. In jedem Fall wird vor Durchführung der Messung das jeweilige Anregungsmittel hinzugegeben, bei dem es nicht wesentlich ist, in welcher Art von Lösungsmittel, sofern erforderlich, es vorliegt. Bevorzugt wird ein wäßriges Medium, insbesondere ein alkalisches, in dem das Anregungsmittel enthalten ist.

Oben wurde ein Vorgehen geschildert, bei dem »indirekt« die chemolumineszierende Markierungssubstanz an den jeweiligen Reaktionspartner einer serologischen Reaktion gebunden wird. Daneben besteht aber auch noch die Möglichkeit, ein »direktes« Assay anzuwenden. Dabei wird entweder ein Antigen oder ein Antikörper vorgegeben. In Abhängigkeit vom Vorgabematerial wird ein Antikörper- bzw. Antigen-Konjugat (mit der chemolumineszierenden Markierungssubstanz) hinzugegeben und ein chemolumineszierender Komplex gebildet, der anschließend isoliert wird. Danach folgt die Chemolumeneszenzmessung nach Zugabe des Anregungsmittels.

Mit großem Vorteil läßt sich das erfindungsgemäße Verfahren auch auf der Grundlage eines Sandwich-Assays durchführen. Ein Sandwich-Assay kann grundsätzlich sowohl im Rahmen eines

Flüssigphasen- als auch eines Festphasenassays angewandt werden. Das wesentliche Kennzeichen eines im Verlaufe eines Sandwich-Assays gebildeten chemolumineszierenden Konjugats ist darin zu sehen, daß in dem bezüglich der Chemolumineszenz gemessenen Komplex der nachzuweisende Reaktionspartner in Form eines Antigens oder eines Antikörpers sowohl mit seinem konjugierten als auch mit seinem nicht-konjugierten Reaktionspartner reagiert hat. Bei Außerachtlassen der gekoppelten Markierungssubstanz handelt es sich also um einen symmetrisch aufgebauten Komplex, bei dem die zentrale Reaktionskomponente das Antigen oder der Antikörper ist, die nachzuweisen ist. Der vorgegebene Reaktionspartner bzw. die vorgegebene Reaktionskomponente tritt an zwei Seiten dieser zentralen Reaktionskomponenten damit in Wechselwirkung.

Dem erfindungsgemäßen Gedanken sollen jedoch auch übliche kompetitive Bindungsassays untergeordnet werden. Bei einem derartigen Assay liegt ein Reaktionspartner einer serologischen Reaktion einerseits markiert und andererseits nicht markiert vor. Diese beiden Reaktionspartner treten mit einem entsprechenden anderen Reaktionspartner in Wechselwirkung. So kann es sich bei dem ersten Reaktionspartner (markiert oder unmarkiert) um ein Antigen handeln. Dann ist der zweite Reaktionspartner ein Antikörper. Erster markierter Reaktionspartner und zweiter Reaktionspartner (Antigen bzw. Antikörper) sind mengenmäßig vorgegeben. Nach Ablauf der serologischen Reaktion wird entweder der nicht gebundene Teil des markierten ersten Reaktionspartners oder der gebildete Komplex in üblicher Weise nach Zugabe des Anregungsmittels gemessen. Diese Technik ist allgemein bekannt (siehe US-PS 4 238 195 a.a.O.). Ihre Variationen sind demzufolge dem Fachmann geläufig.

Die Chemolumineszenz kann mit handelsüblichen Photometern gemessen werden. Dabei läßt sich so vorgehen, daß mit vorgegebenen bekannten Mengen an Antigen und Antikörper eine Eichkurve ermittelt wird. Mit einer unbekannten Substanz (sei es ein Antigen oder ein Antikörper) ermittelte Werte werden dann quantitativ unter Zugrundelegung der Eichkurve ausgewertet.

Des weiteren ist es auch möglich, wenngleich dieses unter halbquantitativen Gesichtspunkten zu sehen ist, einen Vergleich bezüglich der Empfindlichkeit mit anderen bekannten Assays zu ziehen. Das bisher als besonders nachweisempfindlich angesehene Radioimmunoassay, mit den vorstehend geschilderten Nachteilen behaftet, liegt in der Mehrzahl der Anwendungsgebiete bezüglich der Empfindlichkeit weit unter derjenigen des erfindungsgemäßen Verfahrens. So wurde zum Beispiel der Antikörpertiter eines Hyperimmunserums gegen Rinderserumalbumin, hergestellt im Kaninchen, mit 1 : 4069 im Radioimmunoassay ermittelt, während erfindungsgemäß ($H_2O_2$/Chloramin — FITC) eine Empfindlichkeit von 1 : 32788 (!) erzielbar ist. Hierbei handelt es sich also um eine nahezu 8mal so große Empfindlichkeit. Andere übliche Assays waren weitaus weniger empfindlich als die oben verglichenen. So wurden folgende Antikörpertiter mit bekannten Assays ermittelt:

| | |
|---|---|
| im Nephelometer | 1 : 16 |
| beim Immunodiffusionstest | 1 : 64 |
| in der Komplementbindungsreaktion | 1 : 128 und |
| im ELISA | 1 : 512 |

Die mit der Erfindung erzielbaren Vorteile sind insbesondere darin zu sehen, daß die jeweils verwendete chemolumineszierende Markierungssubstanz außerordentlich einfach an einen Reaktionspartner einer serologischen Reaktion, nämlich in Form von Antigenen und Antikörpern bzw. Bindeproteinen, zu binden ist. Zudem sind die erfindungsgemäß in Betracht kommenden Markierungssubstanzen wie auch das Anregungsmittel einfach aufgebaut und für das mit dem erfindungsgemäßen Verfahren befaßte Personal, insbesondere bezüglich der Freisetzung von giftigen Verbindungen, unbedenklich. Die damit hergestellten Konjugate beziehungsweise Komplexe sind relativ stabil, was für die Verfahrensführung von Vorteil ist.

Der mit der Erfindung erzielbare technische Erfolg muß als außergewöhnlich überraschend angesehen werden. So zeigt zum Beispiel das Fluoreszein beziehungsweise dessen Derivate nach der Bindung an einen der Reaktionspartner einer serologischen Reaktion eine wesentlich höhere Empfindlichkeit als die entsprechende ungebundene Verbindung. Die Zahl der ausgesandten Photonen des chemolumineszierenden Konjugats bzw. des chemolumineszierenden Komplexes ist etwa 1000mal höher als die Zahl der Photonen, die von der jeweils ungebundenen Substanz ausgesandt werden. In diesem Zusammenhang ist es von Bedeutung, daß in Journal of Physical Chemistry, Vol. 78, Nr. 17, S. 1681—1683 (1979) ausdrücklich darauf hingewiesen wird, daß zur Erzielung einer meßbaren Photonenausbeute eine unverhältnismäßig große Menge an Fluoreszein oder Fluoreszeinisothiocyanat eingesetzt werden muß. Dieses hat die Fachwelt bisher davon abgehalten, Fluoreszein bzw. seine Derivate im praktischen Rahmen zur Durchführung serologischer Assays auf der Grundlage einer Chemolumineszenzmessung heranzuziehen.

Nachfolgend soll die Erfindung noch näher anhand von Beispielen erläutert werden.

Beispiel 1

Bestimmung der Nachweisgrenzen von reinem Fluoreszeinisothiocyanat (FITC) und Bestimmung der Nachweisgrenze eines FITC-Konjugats

Um die Nachweisgrenze von reinem FITC zu bestimmen, wurde 1 mg FITC in 10 ml destilliertem Wasser aufgelöst. Anschließend wurde die Extinktion einer 1 : 100-Verdünnung bei 495 nm im Photometer gemessen. Von der Ausgangslösung wurden weiterhin 10 Verdünnungen (5er Schritte) angelegt und jeweils 20 µl davon in einem handelsüblichen Photometer gemessen. Zu Beginn der Messung wurden 100 µl einer 2gew.-%igen Calciumhypochloritlösung in die zu messende Probe injiziert. Das Ergebnis der Meßreihen ergibt sich aus der folgenden Zahlenreihe.

| Menge an FITC in ng/ml | gemessene Photonen/sec |
|---|---|
| 20 000 | 18 101 |
| 4 000 | 8 485 |
| 800 | 2 748 |
| 160 | 735 |
| 32 | 394 |

Der Leerwert des Geräts bei Messungen ohne FITC lag unter diesen Bedingungen im Höchstfall bei 380 Photonen/sec. Daher liegt die Nachweisgrenze für reines FITC bei 32 ng/ml.

Um die Nachweisgrenze von an Protein gebundenem FITC zu bestimmen, wurde das FITC an Immunglobolin in G von der Ziege nach der Methode von B. T. Wood, S. H. Thomson und G. Goldstein, beschrieben in Journal of Immunology 95 (1964), S. 225, gebunden. Von dem derartig hergestellten chemolumineszierenden Konjugat wurde ebenfalls die Extinktion bei 495 nm im Photometer gemessen und so die Menge an gebundenem FITC bestimmt. Anschließend wurde ebenfalls eine Verdünnungsreihe von 10 Verdünnungen angelegt und die Chemolumineszenz einer 20-µl-Probe jeder Verdünnung gemessen. Das Ergebnis der Meßreihe ist in der folgenden Zahlenreihe dargestellt.

| Menge an FITC in ng/ml | gemessene Photonen/sec |
|---|---|
| 1000 | 547 982 |
| 200 | 112 895 |
| 40 | 34 683 |
| 8 | 9 914 |
| 1,6 | 3 835 |
| 0,32 | 1 513 |
| 0,064 | 822 |
| 0,0128 | 378 |

Die Nachweisgrenze für das FITC-Protein-Konjugat lag demzufolge bei 0,064 ng/ml. Daraus ergibt es sich, daß die FITC-Konjugate außergewöhnlich gut für den Nachweis von Antigenen, Antikörpern bzw. deren Komplexen über eine serologische Reaktion mittels Chemolumineszenz geeignet sind.

## Beispiel 2

### Photonenausbeuten bei der Verwendung von Methylenblau oder Thionin als Markierungssubstanz

Methylenblau und Thionin wurden in einem Volumen von jeweils 20 µl in den aus der nachfolgenden Tabelle ersichtlichen Mengen vorgegeben. Die Anregung von Methylenblau erfolgte durch eine Zugabe von jeweils 100 µl einer wäßrigen Chloramin T-Lösung (16 mg/ml) und 100 µl einer 10vol.-%igen Wasserstoffperoxidlösung. Die Anregung von Thionin erfolgte durch eine Zugabe von 20 µl einer wäßrigen Chloramin T-Lösung (20 mg/ml) und 100 µl einer 5vol.-%igen Wasserstoffperoxidlösung. Dabei wurden bei einer Reaktionszeit von 30 Sekunden die aus der nachfolgenden Tabelle ersichtlichen vorzüglichen Photonenausbeuten gemessen.

| Markierungs-substanz | Menge | Photonen/30 sec |
|---|---|---|
| Methylenblau | 20 ng | 21 963 |
| Methylenblau | 2 ng | 1 237 |
| Methylenblau | 0,2 ng | 0 |
| Thionin | 2000 pg | 990 000 |
| Thionin | 200 pg | 780 000 |
| Thionin | 20 pg | 210 000 |
| Thionin | 2 pg | 33 000 |
| Thionin | 0,2 pg | 10 000 |

Anmerkung: Der Leerwert betrug im Falle des Thionins 1700 Photonen.

Es wurde ferner die Photonenausbeute bei der Verwendung von Thionin, angeregt mit $H_2O_2$/Chloramin T, gemessen, wobei die Konzentration der beiden Bestandteile des kombinierten Anregungsmittels verändert wurde. Dabei wurden jeweils 2 pg Thionin in 20 µl destillierten Wassers vorgegeben. Die ermittelten Ergebnisse sind in der folgenden Tabelle zusammengestellt. Der jeweilige Leerwert ist in Klammern gesetzt.

| | 20 mg/ml CT | 40 mg/ml CT | 60 mg/ml CT | 80 mg/ml CT |
|---|---|---|---|---|
| 30%iges $H_2O_2$ | 15 100 (2 000) | 15 500 (4 300) | 21 000 (8 000) | 25 400 (12 000) |
| 20%iges $H_2O_2$ | 15 600 (1 950) | 23 300 (6 800) | 29 000 (8 300) | 36 000 (15 000) |
| 10%iges $H_2O_2$ | 44 000 (1 700) | 61 800 (7 800) | 65 000 (18 000) | 77 000 (26 000) |
| 5%iges $H_2O_2$ | 76 000 (1 600) | 103 000 (9 100) | 101 000 (24 000) | 121 000 (45 000) |

## Beispiel 3

### Nachweis von Antikörpern gegen Rinderserumalbumin (BSA) in einem Kaninchenserum

An eine flexible Mikrotiterplatte wurde BSA absorbiert. Dazu wurde das BSA in einer Menge von 0,1 mg/ml in einer Lösung, die mit einem Natriumcarbonat-Puffer auf den pH-Wert von 9,6 gepuffert worden war, gelöst. Davon wurden je 100 µl in einem Napf der Mikrotiterplatte pipettiert.

Anschließend wurde die Platte 16 h lang bei 4°C inkubiert. Nach Absaugen und dreimaligem Waschen der Platte mit einer Phosphat-gepufferten Salzlösung eines pH-Wertes von 7,4, die 1% Sorbimacrogollaurat (vgl. Römpp's Chemielexikon, 7. Auflage, 1967, Bd. 6, S. 3711, 1. Sp. unter T. 20) enthält, wurde eine Verdünnungsreihe des Kaninchenserums in die Näpfe gegeben und 90 min lang bei 37°C inkubiert. Nach Absaugen und erneutem dreimaligen Waschen der Platte wurde jeder Napf mit 100 µl eines FITC-markierten Anti-Kaninchenserums inkubiert (90 min bei 37°C). Nach erneutem dreimaligen Waschen wurden die einzelnen Näpfe ausgeschnitten und die Chemolumineszenz in einem handelsüblichen Photometer gemessen. Dieses Mal wurde die Chemolumineszenz durch Zugabe von 100 µl $H_2O_2$ (30%ig) und 100 µl Chloramin (79 mg/ml) ausgelöst. Das Ergebnis der Messung ergibt sich aus der Figur. Die Zahlenwerte an der Ordinate geben die Anzahl der gemessenen Photonen an. Die Zahlenwerte an der Abszisse drücken den Verdünnungsgrad des Kaninchenhyperimmunserums aus, während es sich bei dem Leerwert um einen Wert handelt, der in spezieller Weise ermittelt wurde. So wurden von mehreren Leerwerten die Durchschnittsleerwerte $\bar{X}$ ermittelt und zu diesem das Produkt aus $2{,}58 \times \sigma$ addiert, wobei der Faktor $\sigma$ die Standardabweichung darstellt. Die Figur zeigt, daß der Grenztiter des Kaninchenserums bei etwa 1 : 32788 liegt. Das gleiche Kaninchenserum hatte im Festphasenradioimmunoassay einen Grenztiter von 1 : 512.

Gleichermaßen gute Ergebnisse wurden erzielt, wenn das Anti-Kaninchenserum mit Methylenblau oder mit Thionin, das mit Hilfe von (N-Äthyl-N'-(3-dimethylamino-propyl)-carbodiimid-Hydrochlorid gekoppelt war, markiert wurde.

## Patentansprüche

1. Verfahren zur quantitativen und qualitativen Bestimmung von Antigenen, Antikörpern und deren Komplexen mittels einer chemolumineszierenden Markierungssubstanz und einem Anregungsmittel hierfür unter Anwendung an sich bekannter heterogener Fest- oder Flüssigphasenassays, dadurch gekennzeichnet, daß eine der folgenden Kombinationen aus Anregungsmittel und Markierungssubstanz verwendet wird:

   $H_2O_2$/Chloramin bzw. -derivat und Fluoreszein bzw. -derivat,
   $H_2O_2$/Chloramin bzw. -derivat und Methylenblau bzw. -derivat,
   $H_2O_2$/Chloramin bzw. -derivat und Thionin- bzw. -derivat,
   Calciumhypochlorit und Fluoreszein bzw. -derivat.

2. Verfahren nach Anspruch 1, dadurch gekennzeichnet, daß $H_2O_2$/Chloramin T als Anregungsmittel verwendet wird.
3. Verfahren nach Anspruch 1 oder 2, dadurch gekennzeichnet, daß als Anregungsmittel eine Mischung aus gleichen Volumina einer 0,5- bis 30vol.-prozentualen wäßrigen Wasserstoffperoxidlösung und einer 0,1- bis 8gew.-prozentualen Lösung an Chloramin T verwendet wird.
4. Verfahren nach einem der Ansprüche 1 bis 3, dadurch gekennzeichnet, daß ein Sandwichassay angewandt wird.
5. Verfahren nach einem der Ansprüche 1 oder 4, dadurch gekennzeichnet, daß Calciumhypochlorit in einer 0,2- bis 10gew.-%igen wäßrigen Lösung zur Messung der Chemolumineszenz verwendet wird.
6. Verfahren nach einem der Ansprüche 1 bis 5, dadurch gekennzeichnet, daß als Fluoreszeinderivat Fluoreszeinisothiocyanat und/oder Fluoreszeinisocyanat verwendet werden.
7. Verfahren nach einem der Ansprüche 1 bis 6, dadurch gekennzeichnet, daß ein Hepatitis-B-Antigen oder Antikörper gegen Herpes- oder Toga-Viren bestimmt werden.

## Claims

1. A method for the quantitative and qualitative determination of antigens, antibodies and complexes thereof by means of a chemoluminescent marking substance and a stimulant therefor, use being made of heterogenous solid or liquid assays known per se, characterized in that one of the following combinations of stimulant and marking substance is used:

   $H_2O_2$/chloramine or a derivative thereof and fluorescein or a derivative thereof,
   $H_2O_2$/chloramine or a derivative thereof and methylene blue or a derivative thereof,
   $H_2O_2$/chloramine or a derivative thereof and thionine or a derivative thereof,
   calcium hypochlorite and fluorescein or a derivative thereof.

2. A method according to claim 1, characterized in that $H_2O_2$/chloramine T is used as the stimulant.
3. A method according to claim 1 or claim 2, characterized in that a mixture of the same volumes of a 0.5 to 30 per cent-vol. aqueous hydrogen peroxide solution and a 0.1 to 8 per cent by weight solution of chloramine T is used.
4. A method according to any one of claims 1 to 3, characterized in that a sandwich assay is used.

5. A method according to claim 1 or claim 4, characterized in that the calcium hypochlorite in a 0.2 to 10 per cent by weight aqueous solution is used for measuring the chemoluminescence.
6. A method according to any one of claims 1 to 5, characterized in that fluorescein isothiocyanate and/or fluorescein isothiocyanate are used as the fluorescein derivative.
7. A method according to any one of claims 1 to 6, characterized in that a hepatitits-B antigen or antibody against herpes viruses or Toga viruses is determined.

**Revendications**

1. Procédé d'analyse quantitative et qualitative d'antigènes, d'anticorps et de leurs complexes au moyen d'une substance de marquage chimioluminescente et d'un agent d'activation de celle-ci en utilisant des essais hétérogènes en phase solide ou liquide connus en soi, caractérisé en ce qu'on utilise l'une des combinaisons suivantes de l'agent d'activation et de la substance de marquage:

$H_2O_2$/chloramine ou dérivé et fluorescéine ou dérivé;
$H_2O_2$/chloramine ou dérivé et bleu de méthylène ou dérivé;
$H_2O_2$/chloramine ou dérivé et thionine ou dérivé;
hypochlorite de calcium et fluorescéine ou dérivé.

2. Procédé selon la revendication 1, caractérisé en ce qu'on utilise, comme agent d'activation, la combinaison $H_2O_2$/chloramine T.
3. Procédé selon la revendication 1 ou 2, caractérisé en ce qu'on utilise comme agent d'activation, un mélange de volumes identiques d'une solution aqueuse d'eau oxygénée à 0,5 à 30% en volume et d'une solution à 0,1 à 8% en poids de chloramine T.
4. Procédé selon l'une des revendications 1 à 3, caractérisé en ce qu'on utilise un essai sandwich.
5. Procédé selon la revendication 1 ou 4, caractérisé en ce qu'on utilise de l'hypochlorite de calcium dans une solution aqueuse à 0,2 à 10% en poids pour mesurer la chimioluminescence.
6. Procédé selon l'une des revendications 1 à 5, caractérisé en ce qu'on utilise, comme dérivé de la fluorescéine, l'isothiocyanate de fluorescéine et/ou l'isocyanate de fluorescéine.
7. Procédé selon l'une des revendications 1 à 6, caractérisé en ce qu'on décèle un antigène d'hépatite B ou un anticorps contre le virus de l'herpès ou le togavirus.

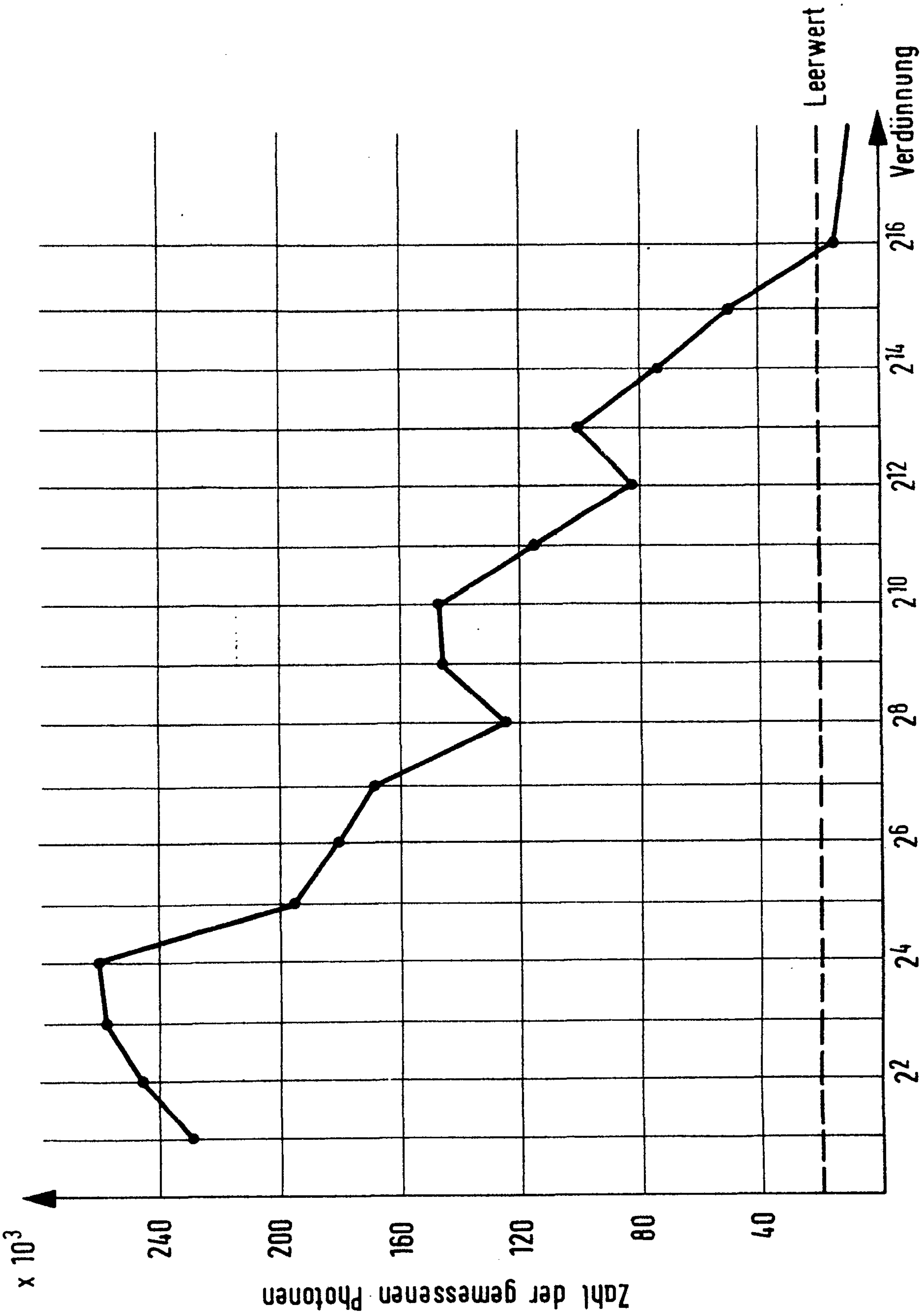

Leerwert
Verdünnung
2^16
2^14
2^12
2^10
2^8
2^6
2^4
2^2
x 10^3
240
200
160
120
80
40
Zahl der gemessenen Photonen